# EUROPÄISCHE PATENTSCHRIFT

(11) **EP 2 839 826 B1**
(45) Veröffentlichungstag und Bekanntmachung des Hinweises auf die Patenterteilung: **27.09.2017**
(21) Anmeldenummer: 14171064.0
(22) Anmeldetag: 04.06.2014
(51) Int. Cl.: A61K 8/29, A61Q 17/04, A61K 8/02, A61K 8/06, A61K 8/31

(54) **Octocrylenfreies Sonnenschutzmittel**
Octocrylene-free sunscreen
Produit de protection contre le soleil sans octocrilène

(30) Priorität: 04.07.2013 DE 102013213174
(43) Veröffentlichungstag der Anmeldung: 25.02.2015
(73) Patentinhaber: Beiersdorf AG, 20253 Hamburg (DE)
(72) Erfinder: Skubsch, Kerstin, 25497 Prisdorf (DE); Nielsen, Jens, 22529 Hamburg (DE); Tesch, Mirko, 22303 Hamburg (DE)

(56) Entgegenhaltungen:
- DE-A1-102004 002 998
- DE-A1-102008 028 665
- US-A1- 2006 008 426

## Beschreibung

Die vorliegende Erfindung betrifft eine kosmetische Zubereitung enthaltend 4-(tert.-Butyl)-4'-methoxydibenzoylmethan und Titandioxid-Partikel in einer Primär-Partikelgrößenverteilung, die mindestens zwei Maxima aufweist, dadurch gekennzeichnet, dass die Zubereitung frei ist von Ethylhexylsalicylat, 2-Ethylhexyl-2-cyano-3,3-diphenylacrylat, Homomenthylsalicylat, 4-Methoxyzimtsäure(2-ethylhexyl)ester und 3-(4-Methylbenzyliden)campher.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Der wohl am häufigsten eingesetzte UV-A-Filter in kosmetischen Zubereitungen ist das 4-(tert.-Butyl)-4'-methoxydibenzoylmethan. Dieses hat jedoch den bekannten Nachteil insbesondere in unpolaren Ölphasen nicht besonders photostabil zu sein, sondern sich unter der Einwirkung von UV-Licht zu zersetzen. Um dieses bekannte Phänomen zu verhindern bzw. zu unterdrücken, werden nach dem Stand der Technik 4-(tert.-Butyl)-4'-methoxydibenzoylmethan haltigen Zubereitungen weitere öllösliche UV-Filter zugesetzt, insbesondere 2-Ethylhexyl-2-cyano-3,3-diphenylacrylat (Octocrylen) aber auch Homomenthylsalicylat, 4-Methoxyzimtsäure(2-ethylhexyl)ester, Ethylhexylsalicylat und Kampferderivate wie 3-(4-Methylbenzyliden)campher. Diese stabilisieren dann den UV-A-Filter in der Zubereitung.

Der Nachteil des Standes der Technik besteht nun in dem Umstand, dass der Einsatz dieser UV-Filter, trotz ihrer Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass diese UV-Filter möglicherweise hormonell wirksam sein könnten. Auch wenn, trotz des jahrzehntelangen weltweiten Einsatzes dieser UV-Filter in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Ein weiterer Nachteil des Standes der Technik besteht in dem Umstand, dass eine Reihe von weiteren Inhaltsstoffen in Sonnenschutzmitteln photolabil sind und/oder unter thermischer Belastung zerstört werden oder sich verflüchtigen. Da Sonnenschutzmittel jedoch ihrem Zweck entsprechend starker UV-Strahlung und Wärme ausgesetzt sind, ist die Thermo- und Photostabilität eine wichtige Voraussetzung für die Wirksamkeit während der gesamten Anwendungsdauer. Problematisch am Stande der Technik ist insbesondere, dass die Zubereitungen aufgrund dieser Phänomene mit zunehmender Anwendungsdauer ihren Geruch ändern. Der frische Duft zu Beginn der Anwendung weicht dann mit zunehmender Anwendungsdauer einem muffigen, ranzigen oder nach "Maggi"-Würze riechenden Duft. Ein solcher "Duftwandel" führt dann dazu, dass die Verbraucher den Einsatz von Sonnenschutzmitteln eher meiden.

Es war daher die Aufgabe der vorliegenden Erfindung, die Nachteile des Standes der Technik zu beseitigen und ein geruchsstabiles Sonnenschutzmittel zu entwickeln, bei dem der photochemische Abbau von 4-(tert.-Butyl)-4'-methoxydibenzoylmethan unterdrückt wird, ohne dass die herkömmlichen Stabilisatoren eingesetzt werden.

Überraschend gelöst werden die Aufgaben durch eine kosmetische Zubereitung enthaltend
a) 4-(tert.-Butyi)-4'-methoxydibenzoylmethan und
b)Titandioxid-Partikel in einer Primär-Partikelgrößenverteilung, die mindestens zwei Maxima aufweist, dadurch gekennzeichnet, dass die Zubereitung frei ist von Ethylhexylsalicylat, 2-Ethylhexyl-2-cyano-3,3-diphenylacrylat, Homomenthylsalicylat, 4-Methoxyzimtsäure(2-ethylhexyl)ester und 3-(4-Methylbenzyliden)campher.

Zwar kennt der Stand der Technik die DE 102008028665.6, DE 102004002998.9 und die US 2006/0008426 doch konnten diese Schriften nicht den Weg zur vorliegenden Erfindung weisen.

Unter Primärpartikeln können erfindungsgemäß die Partikel im Quellenzustand verstanden werden, d. h. in geringem zeitlichen und räumlichen Abstand von der Quelle. Das heißt in der strengen Definition nur unmittelbar nach der Nukleation und vor dem Einsetzen von Agglomerationsvargängen. Die Wert der Primärpartikel können mit verschiedenen Methoden ermittelt werden: Erfindungsgemäß bevorzugt ist die Bestimmung mittels Transmissionselektronenmikroskopie.

Erfindungsgemäß vorteilhaft haben die Primärpartikel
eine mittlere Breite von 7-9 nm und/oder eine Länge von ca. 10-60 nm (Diese Werte wurden mittels Transmissionselektronenmikroskopie ermittelt.).

Es ist erfindungsgemäß vorteilhaft, wenn in der erfindungsgemäßen Zubereitung zwei Titandioxide A und B enthalten sind, die sich in ihrer Primär-Partikelgröße voneinander unterscheiden.

Erfindungsgemäß vorteilhafte Ausführungsformen der vorliegenden Erfindung sind dadurch gekennzeichnet, dass ein Teil der Titandioxid-Partikel (A oder B) mit Alumina und Stearinsäure oberflächenbeschichtet ist.

Ferner sind erfindungsgemäß voreilhafte Ausführungsformen der vorliegenden Erfindung, dadurch gekennzeichnet, dass die Zubereitung Titandioxid-Partikel mit unterschiedlicher Oberflächenbeschichtung enthält.

Vorteilhafte Ausführungsformen der vorliegenden Erfindung sind auch dadurch gekennzeichnet, dass ein Teil der Titandioxid-Partikel (A oder B) mit Dimethoxycaprylylsilan oberflächenbeschichtet ist.

Eine alternative, erfindungsgemäß vorteilhafte Ausführungsform der vorliegenden Erfindung ist dadurch gekennzeichnet, dass ein Teil der Titandioxid-Partikel (A oder B) mit Aluminium Hydroxid und/oder Silica und/oder Stearinsäure oberflächenbeschichtet ist.

Es ist erfindungsgemäß vorteilhaft, wenn die durchschnittliche Primär-Partikelgröße der Titandioxid-Partikel kleiner als 100 nm beträgt.

Es ist erfindungsgemäß von Vorteil, wenn die erfindungsgemäße Zubereitung Titandioxid in einer Gesamtmenge von 0,5 bis 20 Gewichts-%, bezogen auf das Gesamtgewicht der Zubereitung, enthält.

Es ist erfindungsgemäß bevorzugt, wenn die erfindungsgemäße Zubereitung Titandioxid in einer Gesamtmenge von 1 bis 7 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die erfindungsgemäße Zubereitung 4-(tert.-Butyl)-4'-methoxydibenzoylmethan in einer Konzentration von 0,1 bis 6 Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die erfindungsgemäße Zubereitung 4-(tert.-Butyl)-4'-methoxydibenzoylmethan in einer Konzentration von 0,5 bis 5 Gewichts-%, bezogen auf das Gesamtgewicht der Zubereitung, enthält.

Es ist erfindungsgemäß vorteilhaft, wenn die erfindungsgemäße Zubereitung in Form einer Emulsion oder Dispersion, bevorzugt in Form einer Emulsion und besonders bevorzugt in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Es ist erfindungsgemäß von Vorteil, wenn die erfindungsgemäße Zubereitung weitere UV-Filter, insbesondere UV-B-Filter enthält. Diese können erfindungsgemäß vorteilhaft aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Terephthalidendicampher-sulfonsäure; 4-(Dimethylamino)-benzoesäure(2-ethylhexyl)ester; 4-(Dimethylamino)-benzoesäureamylester; 4-Methoxybenzalmalon-säuredi(2-ethylhexyl)-ester; 4-Methoxyzimtsäureisoamylester; 2-(4'-Diethylamino-2'-hydoxybenzoyl)-benzoesäurehexylester, 2-Ethylhexyl-2-hydroxy-benzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamido-triazon (INCI: Diethylhexyl-Butamidotriazone); 2,4-bis-[5-1(dimethylpropyl)benz-oxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2, 4-B is-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tribiphenyl-4-yl-1,3,5-triazin; Zinkoxid und/oder Merocyanine gewählt werden.

Es ist erfindungsgemäß von Vorteil, wenn die erfindungsgemäße Zubereitung frei ist von Polyethylenglycol, Polyethylenglycolethern und Polyethylenglycolestern (sogenannten PEG-Derivaten).

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, β-Alanin, Tocopherol, Tocopherolacetat und/oder Licochalcon A, enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Erfindungsgemäß vorteilhafte Ausführungsformen der vorliegenden Erfindung sind dadurch gekennzeichnet, dass die Zubereitung frei ist von Parabenen, Benzethoniumchlorid, Piroctone Olamine, Lauroylarginat, Benzoesäure, Sorbinsäure, Methylisothiazolinon, Chlormethylisothiazolinon, Bronopol, Benzalkoniumchloride, Formaldehydabspalter, Salicylsäure, Triclosan, Dehydroacetsäure , DMDM Hydantoin, Chlorphenesin, IPBC.

Vielmehr ist es erfindungsgemäß vorteilhaft, wenn die erfindungsgemäße Zubereitung Ethanol enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen),

Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum und/oder Polyethylen, Nylon, Silica Diemthyl Silyate

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Komplexbildner (wie z.B. EDTA-Salze), Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 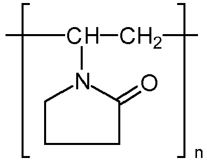 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PlB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Eine erfindungsgemäß vorteilhafte Ausführungsform stellen Zubereitungen dar, die keine Parfümstoffe enthalten.

Erfindungsgemäß ist die Verwendung der erfindungsgemäßen Zubereitung zum Schutz vor Hautalterung (insbesondere zum Schutz vor UV-bedingter Hautalterung) sowie als Sonnenschutzmittel.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Zusammensetzung** | **Beispiel A Gew.%** |
|---|---|
| Titaniumdioxid + Alumina + Stearinsäure (Solaveil XT-P1) Primärpartikelgröße | 3 |
| Ethylhexylglycerin | 0,5 |
| Dibutyl Adipat | 3 |
| Cetearyl Alkohol | 1 |
| Butylene Glycol Dicaprylat/Dicaprat | 5 |
| Phenethyl Benzoat | 4,5 |
| C12-15 Alkyl Benzoat | 6 |
| Glyceryl Stearat SE | 1 |
| Natrium Cetearyl Sulfat | 0,15 |
| VP/Hexadecene Copolymer | 0,5 |
| Glycerin | 7,5 |
| Neutralisationsmittel | q.s. |
| Methylpropandiol | 3 |
| Acrylat/C10-30 Alkyl Acrylat Crosspolymer | 0,1 |
| Xanthan Gummi | 0,4 |
| VE Wasser | ad 100 |
| Ethanol vergällt | 4 |
| Trisodium EDTA | 1 |
| Bis-Ethylhexyloxyphenol Methoxyphenyl Triazin | 4 |
| Ethylhexyl Triazin | 3 |
| Butyl Methoxydibenzoylmethan | 4,75 |
| Titaniumdioxid + Trimethoxycaprylylsilan (Tego Sun T 805) Primarpartikelgröße 15 nm | 3 |
| Phenylbenzimidazolsulfonsäure | 1 |
| | |
| Colipa Ratio kalkuliert mit BASF-Calculator | 3,5 |
| Colipa Ratio Messwert | 2,2 |
| SPF | 60 |

| | **1** | **2** | **3** | **4** | **5** | **6** |
|---|---|---|---|---|---|---|
| Natriumstearylglutamate | | | 0,3 | | 0,2 | 0,3 |
| Glyceryl Stearat SE | 1 | 1 | | | | |
| Natrium Cetearyl Sulfat | 0,1 | 0,1 | | | | |
| Cetearylalkohol | 1 | 1 | | | 1 | |
| Polyglyceryl-3 Methylglucose Distearate | | | | 3 | | |
| Sorbitan Stearate | | | | 1 | | |
| Acrylates/C₁₀₋₃₀ Alkyl Acrylat Crosspolymer | 0,1 | 0,1 | 0,3 | | | 0,1 |
| Carbomer | | | 0,1 | | 0,4 | |
| Xanthan Gummi | 0,3 | 0,3 | | 0,5 | | 0,4 |
| C₁₂₋₁₅ Alkyl Benzoat | 5 | 5 | | 4 | | |
| Butylenglycol Dicaprylat/Dicaprat | 5 | 5 | 8 | | | |
| Methylpropandiol | 3 | 3 | | | | 5 |
| Dibutyladipat | | | 4 | 3 | | |
| Ethylhexylglycerin | 0,2 | | | | 0,5 | 0,5 |
| 1,2-Hexandiol | | | 1 | | 0,2 | 0,3 |
| Di-C12-13 Alkyl Tartrate | | | | 5 | | |
| PVP Hexadecen Copolymer | | 0,5 | 0,5 | | | |
| Glycerin | 5 | 10 | 3 | 7 | 5 | 5 |
| Alkohol denat. | 4 | 6 | 2 | | 4 | 8 |
| Titanium Dioxid+Aluminum Hydroxid+ Dimethicone/Methicon Copolymer; Partikelgröße 15 nm | | | 2 | 1 | 2 | |
| Titanium Dioxid+Dimethicon/Methicon Copolymer; Partikelgröße 80 | | | 2 | | | |
| Titanium Dioxid + Trimethoxycaprylylsilan, Partikelgröße 15nm | 1,5 | 2 | | | | 4 |
| Titaniumdioxid + Alumina + Stearinsäure (Solaveil XT-P1) Primärpartikelgröße | 1,5 | 3 | | 1 | 0,5 | 1 |
| Bis-Ethylhexyloxyphenol Methoxyphenyltriazin | 2 | 2 | | 3 | | |
| Ethylhexyltriazin | | 1 | 3 | | | |
| Diethylhexyl Butamido Triazone | | | | | | 2 |
| Butyl Methoxydibenzoylmethan | 3 | 3 | 4 | 4 | 2 | 5 |
| Phenylbenzimidazol Sulfonsäure | 0,5 | | | | | |
| Ethylhexylsalicylat | | | | 4 | | 5 |
| C18-38 Alkyl Hydroxystearoyl Stearate | 1 | 1 | | | | |
| 2,2'-Methylen-bis-(6-(2H-benzotriazol-2-yl)-4-(1,1,3,3-tetramethylbutyl)-phenol) | | | | 4 | | |
| 2,4,6-Tris-(biphenyl)-1,3,5-triazin | | | | | 3 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Konservierungsmittel | q.s. | q.s. | q.s. | q.s. | q.s. | q.s. |
| Neutralisationsmittel (z.B. NaOH) | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Zubereitung enthaltend
a) 4-(tert.-Butyl)-4'-methoxydibenzoylmethan und
b)Titandioxid-Partikel in einer Primär-Partikelgrößenverteilung, die mindestens zwei Maxima aufweist, **dadurch gekennzeichnet, dass** die Zubereitung frei ist von Ethylhexylsalicylat, 2-Ethylhexyl-2-cyano-3,3-diphenylacrylat, Homomenthylsalicylat, 4-Methoxyzimtsäure(2-ethylhexyl)ester und 3-(4-Methylbenzyliden)campher.

2. Kosmetische Zubereitung nach Anspruch 1, **dadurch gekennzeichnet, dass** in der Zubereitung zwei Titandioxide A und B enthalten sind, die sich in ihrer Primär-Partikelgröße voneinander unterscheiden.

3. Kosmetische Zubereitung nach einem der vorhergehenden Ansprüche, **dadurch gekennzeichnet, dass** die Zubereitung Titandioxid-Partikel mit unterschiedlicher Oberflächenbeschichtung enthält.

4. Kosmetische Zubereitung nach Anspruch 3, **dadurch gekennzeichnet, dass** ein Teil der Titandioxid-Partikel (A oder B) mit Alumina und Stearinsäure oberflächenbeschichtet ist.

5. Kosmetische Zubereitung nach einem der Ansprüche nach einem der Ansprüche 3 oder 4, **dadurch gekennzeichnet, dass** ein Teil der Titandioxid-Partikel (A oder B) mit Dimethoxycaprylylsilan oberflächenbeschichtet ist.

6. Kosmetische Zubereitung nach Anspruch 3, **dadurch gekennzeichnet, dass** ein Teil der Titandioxid-Partikel (A oder B) mit Aluminium Hydroxid und/oder Silica oberflächenbeschichtet ist.

7. Kosmetische Zubereitung nach einem der vorhergehenden Ansprüche, **dadurch gekennzeichnet, dass** die durchschnittliche Primär-Partikelgröße der Titandioxid-Partikel kleiner als 100 nm beträgt.

8. Kosmetische Zubereitung oder Verwendung nach einem der vorhergehenden Ansprüche, **dadurch gekennzeichnet, dass** die Zubereitung in Form einer Emulsion oder Dispersion, bevorzugt in Form einer Emulsion und besonders bevorzugt in Form einer O/W-Emulsion vorliegt.

9. Kosmetische Zubereitung nach einem der vorhergehenden Ansprüche, **dadurch gekennzeichnet, dass** die Zusammensetzung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Suffo-3-bornylidenmethyl)-Benzol und dessen Salze; 4-(2-Oxo-3-bornylidenme-thyl)benzolsulfonsäuresalze; 2-Methyl-5-(2-oxo-3-bornylidenmethyl)sulfonsäuresalze; 2,2'-Methylen-bis-(6-(2H-benzotrrazol-2-yl)-4-(1,1,3,3-tetramethylbutyl)-phenol); 2-(2H-benzotriazol-2-yf)-4-methyl-6-[2-methyl-3-[1,3,3,3-tetramethyl-1-[(trimethylsilyl)oxy]disiloxanyl]propyl]-phenol; 3-Benzylidencampher; Terephthalidendicampher-sulfonsäure; 4-(Dimethylamino)-benzoesäure(2-ethylhexyl)ester; 4-(Dimethylamino)-benzoesäureamylester; 4-Methoxybenzalmalon-säuredi(2-ethylhexyl)ester; 4-Methoxyzimtsäureiscamylester; 2-(4'-Diethylamino-2'-hydoxybenzoyl)-benzoesäurehexylester, 2-Ethylhexyl-2-hydroxy-benzoat; Dimethicodiethylbenzalmalonat; 3-(4-(2,2-bis Ethoxycarbonylvinyl)-phe-noxy)propenyl)-methoxysiloxan / aimethylsiloxan - Copolymer; Dioctylbutylamido-triazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexy[oxyphenol Methoxyphenyl Triazin); 2,4,6-Tribiphenyl-4-yl-1,3,5-triazin; Zinkoxid und/oder Merocyanine.

10. Kosmetische Zubereitung nach Ansspruch 1, **dadurch gekennzeichnet, dass** die Zubereitung frei ist von Polyethylenglycol, Polyethylenglycolethern und Polyethylenglycolestern.

11. Kosmetische Zubereitung nach einem der vorhergehenden Ansprüche, **dadurch gekennzeichnet**, das die Zubereitung Titandioxid in einer Gesamtmenge von 0,5 bis 20 Gewichts-%, bezogen auf das Gesamtgewicht der Zubereitung, enthält.

12. Kosmetische Zubereitung nach einem der vorhergehenden Ansprüche, **dadurch gekennzeichnet**, das die Zubereitung 4-(tert.-Butyl)-4'-methoxydibenzoylmethan in einer Konzentration von 0,1 bis 6 Gewichts-%, bezogen auf das Gesamtgewicht der Zubereitung, enthält.

13. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

14. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält,

15. Kosmetische Zubereitung nach einem der vorhergehenden Ansprüche, **dadurch gekennzeichnet, dass** die Zubereitung frei ist von Parabenen, Benzethoniumchlorid, Piroctone Olamine, Lauroylarginat, Benzoesäure, Sorbinsäure, Methylisothiazolinon, Chlormethylisothiazolinon, Bronopol, Benzalkoniumchloride, Formaldehydabspalter, Salicylsäure, Triclosan, Dehydroacetsäure , DMDM Hydantoin, Chlorphenesin, IPBC.

16. Kosmetische Zubereitung nach einem der vorhergehenden Ansprüche, **dadurch gekennzeichnet, dass** die Zubereitung Ethanol enthält.

## Claims

1. Cosmetic preparation comprising
a) 4-(tert-butyl)-4'-methoxydibenzoylmethane and
b) titanium dioxide particles in a primary particle size distribution having at least two maxima, **characterized in that** the preparation is free of ethylhexyl salicylate, 2-ethylhexyl 2-cyano-3,3-diphenylacrylate, homomenthyl salicylate, 2-ethylhexyl 4-methoxycinnamate and 3-(4-methylbenzylidene)camphor.

2. Cosmetic preparation according to Claim 1, **characterized in that** in the preparation two titanium dioxides A and B are present, which differ from each other in their primary particle size.

3. Cosmetic preparation according to either of the preceding claims, **characterized in that** the preparation comprises titanium dioxide particles with different surface coatings.

4. Cosmetic preparation according to Claim 3, **characterized in that** a portion of the titanium dioxide particles (A or B) has been surface-coated with alumina and stearic acid.

5. Cosmetic preparation according to any of the claims according to either of Claims 3 or 4, **characterized in that** a portion of the titanium dioxide particles (A or B) has been surface-coated with dimethoxycaprylylsilane.

6. Cosmetic preparation according to Claim 3, **characterized in that** a portion of the titanium dioxide particles (A or B) has been surface-coated with aluminium hydroxide and/or silica.

7. Cosmetic preparation according to any of the preceding claims, **characterized in that** the average primary particle size of the titanium dioxide particles is less than 100 nm.

8. Cosmetic preparation or use according to any of the preceding claims, **characterized in that** the preparation is in the form of an emulsion or dispersion, preferably in the form of an emulsion and particularly preferably in the form of an O/W emulsion.

9. Cosmetic preparation according to any of the preceding claims, **characterized in that** the composition comprises one or more further UV filters selected from the group of compounds comprising phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho₋3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; isoamyl 4-methoxycinnamate; hexyl 2-(4'-diethylamino-2'-hydoxybenzoyl)benzoate; 2-ethylhexyl 2-hydroxybenzoate; dimethicodiethylbenzalmalonate; 3-(4-(2,2-bis ethoxycarbonylvinyl)phenoxy)propenyl)methoxysiloxa ne / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4''-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4 bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tribiphenyl-4-yl-1,3,5-triazine; zinc oxide and/or merocyanine.

10. Cosmetic preparation according to Claim 1, **characterized in that** the preparation is free of polyethylene glycol, polyethylene glycol ethers and polyethylene glycol esters.

11. Cosmetic preparation according to any of the preceding claims, **characterized in that** the preparation comprises titanium dioxide in a total amount of 0.5 to 20% by weight, based on the total weight of the preparation.

12. Cosmetic preparation according to any of the preceding claims, **characterized in that** the preparation comprises 4-(tert-butyl)-4'-methoxydibenzoylmethane at a concentration of 0.1 to 6% by weight, based on the total weight of the preparation.

13. Cosmetic preparation according to any of the preceding claims, **characterized in that** the preparation comprises one or more active ingredients selected from the group of compounds comprising glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

14. Cosmetic preparation according to any of the preceding claims, **characterized in that** the preparation comprises propylene glycol, butyelene glycol, 2-methylpropane-,1,3,-diol, 1,2-pentanediol, 1,2-hexanediol, 1,2-octanediol and/or 1,2-decanediol.

15. Cosmetic preparation according to any of the preceding claims, **characterized in that** the preparation is free of parabens, benzethonium chloride, piroctone olamine, lauroyl arginate, benzoic acid, sorbic acid, methylisothiazolinone, chloromethylisothiazolinone, bronopol, benzalkonium chlorides, formaldehyde releasers, salicylic acid, triclosan, dehydroacetic acid, DMDM hydantoin, chlorophenesin, IPBC.

16. Cosmetic preparation according to any of the preceding claims, **characterized in that** the preparation comprises ethanol.

## Revendications

1. Préparation cosmétique contenant :
a) du 4-(tert.-butyl)-4'-méthoxydibenzoylméthane et
b) des particules de dioxyde de titane en une distribution des tailles de particules primaires qui comprend au moins deux maxima, **caractérisée en ce que** la préparation est exempte de salicylate d'éthylhexyle, d'acrylate de 2-éthylhexyl-2-cyano-3,3-diphényle, de salicylate d'homomenthyle, d'ester 2-éthylhexylique de l'acide 4-méthoxycinnamique et de 3-(4-méthylbenzylidène)camphre.

2. Préparation cosmétique selon la revendication 1, **caractérisée en ce que** deux dioxydes de titane A et B sont contenus dans la préparation, qui diffèrent l'un de l'autre par leur taille de particule primaire.

3. Préparation cosmétique selon l'une quelconque des revendications précédentes, **caractérisée en ce que** la préparation contient des particules de dioxyde de titane comprenant un revêtement de surface différent.

4. Préparation cosmétique selon la revendication 3, **caractérisée en ce qu'**une partie des particules de dioxyde de titane (A ou B) est revêtue en surface avec de l'alumine et de l'acide stéarique.

5. Préparation cosmétique selon l'une quelconque des revendications l'une quelconque des revendications 3 ou 4, **caractérisée en ce qu'**une partie des particules de dioxyde de titane (A ou B) est revêtue en surface avec du diméthoxycaprylylsilane.

6. Préparation cosmétique selon la revendication 3, **caractérisée en ce qu'**une partie des particules de dioxyde de titane (A ou B) est revêtue en surface avec de l'hydroxyde d'aluminium et/ou de la silice.

7. Préparation cosmétique selon l'une quelconque des revendications précédentes, **caractérisée en ce que** la taille de particule primaire moyenne des particules de dioxyde de titane est inférieure à 100 nm.

8. Préparation cosmétique ou utilisation selon l'une quelconque des revendications précédentes, **caractérisée en ce que** la préparation se présente sous la forme d'une émulsion ou d'une dispersion, de préférence sous la forme d'une émulsion et de manière particulièrement préférée sous la forme d'une émulsion H/E.

9. Préparation cosmétique selon l'une quelconque des revendications précédentes, **caractérisée en ce que** la composition contient un ou plusieurs filtres UV supplémentaires choisis dans le groupe constitué par les composés sels de l'acide phénylène-1,4-bis-(2-benzimidazyl)-3,3'-5,5'-tétrasulfonique ; sels de l'acide 2-phénylbenzimidazole-5-sulfonique ; 1,4-di (2-oxo-10-sulfo-3 -bornylidène-méthyl) -benzène et ses sels ; sels de l'acide 4-(2-oxo-3-bornylidène-méthyl)benzène-sulfonique ; sels, de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3=tétraméthyl-1-[(triméthyl-silyl)oxy]disiloxanyl]propyl]-phénol ; 3-benzylidène-camphre ; acide téréphtalidène-dicamphre-sulfonique ; ester 2-éthylhexylique de l'acide 4-(diméthylamino)-benzoïque ; ester amylique de l'acide 4-(diméthylamino)-benzoïque ; ester di(2-éthylhexylique) de l'acide 4-méthoxybenzalmalonique ; ester isoamylique de l'acide 4-méthoxycinnamique ; ester hexylique de l'acide 2-(4'-diéthylamino-2'-hydroxybenzoyl)-benzoïque ; 2-hydroxy-benzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine de n° CAS 288254-16-0 ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxy-phényl)-1,3,5-triazine (INCI Bis-Ethylhexyloxyphenol Methoxyphenyl Triazin) ; 2,4,6-tribiphényl-4-yl-1,3,5-triazine ; oxyde de zinc et/ou mérocyanine.

10. Préparation cosmétique selon la revendication 1, **caractérisée en ce que** la préparation est exempte de polyéthylène glycol, d'éthers de polyéthylène glycol et d'esters de polyéthylène glycol.

11. Préparation cosmétique selon l'une quelconque des revendications précédentes, **caractérisée en ce que** la préparation contient du dioxyde de titane en une quantité totale de 0,5 à 20 % en poids, par rapport au poids total de la préparation.

12. Préparation cosmétique selon l'une quelconque des revendications précédentes, **caractérisée en ce que** la préparation contient du 4-(tert.-butyl)-4'-méthoxydibenzoylméthane en une concentration de 0,1 à 6 % en poids, par rapport au poids total de la préparation.

13. Préparation cosmétique selon l'une quelconque des revendications précédentes, **caractérisée en ce que** la préparation contient un ou plusieurs agents actifs choisis dans le groupe constitué par les composés acide glycyrrhétinique, urée, arctiine, acide alpha-liponique, acide folique, phytoène, D-biotine, co-enzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

14. Préparation cosmétique selon l'une quelconque des revendications précédentes, **caractérisée en ce que** la préparation contient du propylène glycol, du butylène glycol, du 2-méthylpropane-1,3-diol, du 1,2-pentanediol, du 1,2-hexandiol, du 1,2-octanediol et/ou du 1,2-décanediol.

15. Préparation cosmétique selon l'une quelconque des revendications précédentes, **caractérisée en ce que** la préparation est exempte de parabènes, de chlorure de benzéthonium, de pyroctone olamine, d'arginate de lauroyle, d'acide benzoïque, d'acide sorbique, de méthylisothiazolinone, de chlorométhylisothiazolinone, de bronopol, de chlorure de benzalkonium, de séparateurs de formaldéhyde, d'acide salicylique, de triclosan, d'acide déshydroacétique, de DMDM-hydantoïne, de chlorphénésine, d'IPBC.

16. Préparation cosmétique selon l'une quelconque des revendications précédentes, **caractérisée en ce que** la préparation contient de l'éthanol.
